# EUROPÄISCHE PATENTSCHRIFT

(11) **EP 2 648 666 B1**
(45) Veröffentlichungstag und Bekanntmachung des Hinweises auf die Patenterteilung: **25.01.2017**
(21) Anmeldenummer: 10795616.1
(22) Anmeldetag: 10.12.2010
(51) Int. Cl.: A61F 9/007, A61F 9/008, G02B 26/12

(54) **LASEREINRICHTUNG, INSBESONDERE FÜR DIE OPHTHALMOLOGISCHE LASERCHIRURGIE**
LASER ARRANGEMENT, IN PARTICULAR FOR OPHTHALMOLOGICAL LASER SURGERY
DISPOSITIF LASER DESTINÉ NOTAMMENT À LA CHIRURGIE LASER OPHTALMOLOGIQUE

(43) Veröffentlichungstag der Anmeldung: 16.10.2013
(73) Patentinhaber: WaveLight GmbH, 91058 Erlangen (DE)
(72) Erfinder: WÖLFEL, Mathias, 91052 Erlangen (DE); Dr. KITTELMANN, Olaf, 14109 Berlin (DE); THUERMER, Daniel, 90443 Nürnberg (DE)
(74) Vertreter: Katérle, Axel
(86) Internationale Anmeldenummer: PCT/EP2010/007532
(87) Internationale Veröffentlichungsnummer: WO 2012/076032

(56) Entgegenhaltungen:
- US-A1- 2002 097 378
- US-A1- 2002 193 704
- US-A1- 2007 173 794
- US-A1- 2009 118 718
- unknown: "PID controller", , 3. Dezember 2010 (2010-12-03), XP000002656445, Gefunden im Internet: URL:http://en.wikipedia.org/w/index.php?ti tle=PID_controller&oldid=400315020 [gefunden am 2011-08-08]

## Beschreibung

Die Erfindung befasst sich mit Lasereinrichtungen und insbesondere mit dem Testen einer Scanfunktion einer Lasereinrichtung.
Lasereinrichtungen, die mit fokussierter Laserstrahlung arbeiten, um totes oder lebendes Gewebe (z.B. humanes Augengewebe) zu bearbeiten, weisen häufig steuerbare Komponenten auf, die eine Scanfunktion ermöglichen. Mit der Scanfunktion kann der Strahlungsfokus präzise auf unterschiedliche Positionen in einer zur Strahlungsausbreitungsrichtung orthogonalen Ebene (transversales Scannen) oder/und auf unterschiedliche Positionen längs der Strahlungsausbreitungsrichtung (longitudinales Scannen) eingestellt werden. Beispiele für Bauteile, die zum Scannen von Laserstrahlung dienen können, sind schwenkbar angeordnete Spiegel, verformbare Spiegel, elektrooptische Kristalle, verschiebbar angeordnete Linsen, brechtkraftvariable Linsen, usw. Wenn im Rahmen dieser Offenbarung von Scankomponenten die Rede ist, so sind damit nicht nur die auf die Laserstrahlung einwirkenden optischen Bauteile gemeint, sondern die Gesamtheit der Komponenten, die zum Scannen der Laserstrahlung benötigt werden und durch elektrische Steuersignale einer elektronischen Steueranordnung beeinflussbar sind. So gehören zu den Scankomponenten im Sinne der Erfindung insbesondere auch die zur Betätigung der optischen Scanbauteile gegebenenfalls nötigen, durch die Steuersignale der Steueranordnung ansteuerbaren Steller. Solche Steller können beispielsweise Galvanometerantriebe, Piezoantriebe, motorische Antriebe, steuerbare Spannungs- oder Stromquellen usw. umfassen. Es versteht sich, dass die vorstehende Aufzählung möglicher optischer Scanbauteile und Steller rein beispielhaft ist und nicht beschränkend zu verstehen ist.
Die Erfindung schlägt eine bevorzugt für die ophthalmologische Laserchirurgie bestimmte und eingerichtete Lasereinrichtung vor, umfassend eine Laserquelle zur Bereitstellung von Laserstrahlung, steuerbare Scankomponenten zur Einstellung einer Fokusposition der Laserstrahlung, Messkomponenten zur Erfassung von Informationen, welche für eine Ist-Position des Strahlungsfokus repräsentativ sind, sowie eine die Laserquelle und die Scankomponenten steuernde Steueranordnung, welche dazu eingerichtet ist, die Durchführung eines Testbetriebslaufs mindestens eines Teils der Scankomponenten bei abgeschalteter Laserquelle nach Maßgabe eines vorgegebenen Test-Scanmusters zu bewirken, wobei das Test-Scanmuster eine Mehrzahl nacheinander anzufahrender diskreter Soll-Fokuspositionen repräsentiert und die Steueranordnung dazu eingerichtet ist, bei jeder der Soll-Fokuspositionen die Scanbewegung der Scankomponenten anzuhalten und in Zuordnung zu jeder der Soll-Fokuspositionen jeweils eine Ist-Fokusposition auf Grundlage der erfassten Informationen der Messkomponenten zu ermitteln. Der Testbetriebslauf ist sozusagen ein Trockenlauf, bei dem die Scanfunktion der Lasereinrichtung überprüft werden kann, ohne dass gleichzeitig Laserstrahlung von der Lasereinrichtung abgegeben wird.

Ein solches System ist aus US2002/0193704 bekannt. Alterungserscheinungen, ein längerer Nichtgebrauch oder Unterbrechungen der Datentransferwege können dazu führen, dass eine von der Steueranordnung befohlene Soll-Einstellung der Scankomponenten (entsprechend einer bestimmten SollPosition des Strahlungsfokus) nicht präzise umgesetzt wird und die tatsächlich erzielte Ist-Einstellung der Scankomponenten von der Soll-Einstellung abweicht. Bei eingeschalteter Laserquelle würde dann die Ist-Position des Strahlungsfokus von der SollPosition abweichen. Bei der Bearbeitung von toter Materie mag dies insofern noch hinnehmbar sein, als die Bearbeitung mit einem neuen Werkstück wiederholt werden kann, wenn sich herausstellt, dass die erste Bearbeitung nicht ausreichend präzise war. Bei lebendem Gewebe, wie beispielsweise humanem Augengewebe, ist eine solche Vorgehensweise aus nachvollziehbaren Gründen nicht gangbar. Die Durchführung eines vorherigen Testbetriebslaufs der Scankomponenten kann Gewissheit darüber verschaffen, dass bei der anschließenden Laserbearbeitung tatsächlich Soll und Ist ausreichend genau beieinander liegen, oder/und sie kann Aufklärung über das Ausmaß etwaiger Abweichungen zwischen Soll und Ist verschaffen, so dass vor der Laserbearbeitung noch geeignete Korrekturmaßnahmen (z.B. Austausch zumindest eines Teils der Scankomponenten, Ermittlung von Korrekturfaktoren und Anpassung der Soll-Werte anhand der Korrekturfaktoren) veranlasst werden können.
Das Test-Scanmuster repräsentiert eine Mehrzahl einzeln anzufahrender diskreter Soll-Fokuspositionen, wobei die Scankomponenten (allgemein: das Scansystem) von der Steueranordnung derart gesteuert werden, dass die von den Scankomponenten bewirkte Scanbewegung an den Soll-Fokuspositionen jeweils stoppt, so dass die Ist-Fokuspositionen im Stillstand gemessen werden. Vorzugsweise wird mit der Messung der Ist-Fokusposition jeweils eine gewisse Zeit (Einschwingzeit) abgewartet, nachdem die Scankomponenten eine Soll-Fokusposition erreicht haben. Diese Einschwingzeit kann beispielsweise in der Größenordnung von einer oder mehreren Millisekunden liegen. Indem die Messung der Ist-Fokuspositionen im Stillstand erfolgt, ist es möglich, die maximal mögliche Messgenauigkeit der Messkomponenten (Messeinrichtung) zu nutzen.

Die Soll-Fokuspositionen sind vorzugsweise so verteilt, dass alle Bereiche des nominell verfügbaren Scanraums abgedeckt sind. Es ist so eine großräumige Vorabprüfung der Positioniergenauigkeit der Scanfunktion in allen anfahrbaren Bereichen des Scanraums möglich. Durch Wahl einer nicht zu großen Anzahl von Soll-Fokuspositionen kann gleichzeitig der Prüfvorgang kurzgehalten werden, was insbesondere ermöglicht, den Testbetriebslauf vor jeder geplanten Laserbearbeitung durchzuführen, ohne hierbei unnötig viel Zeit zu verlieren. Beispielsweise kann es genügen, weniger als 100, besser weniger als 50 und noch besser weniger als 20 Soll-Fokuspositionen vorab festzulegen, die im Rahmen des Testbetriebslaufs angefahren werden sollen.

Es versteht sich, dass der Hinweis auf Soll- und Ist-Fokuspositionen nur der gedanklichen Klarstellung dient, da in dem Testbetriebslauf keine Laserstrahlung auf die Scankomponenten fällt und dementsprechend auch kein Strahlungsfokus vorhanden ist. Bei eingeschalteter Laserquelle würde freilich der dann vorhandene Strahlungsfokus eine Position einnehmen, die durch die Ist-Einstellung der Scankomponenten gegeben ist.

Typischerweise ist der Bereich, innerhalb dessen der Strahlungsfokus verstellt werden kann, durch konstruktive, phyikalische oder/und steuerungstechnische Vorgaben begrenzt. Der Anwender hat so ein vorgegebenes maximales Scanfeld zur Verfügung, das die äußeren Grenzen für die Scanbewegungen des Strahlungsfokus festlegt. Bei Ausführungsformen, die allein transversale Fokusbewegungen ermöglichen, ist das verfügbare Scanfeld demnach eine Transversalfläche. Bei Ausführungsformen dagegen, die transversale und longitudinale Fokusbewegungen ermöglichen, ist das verfügbare Scanfeld ein dreidimensionaler Raum. Transversal kann das verfügbare Scanfeld beispielsweise eine kreisförmige Außengrenze haben. Im dreidimensionalen Fall kann das verfügbare Scanfeld beispielsweise kreiszylindrische Form haben.

Zumindest in manchen Fällen kann es sein, dass das verfügbare Scanfeld bis nahe an seine Grenzen heran für eine Applikation genutzt werden soll. Nicht nur, aber besonders dann ist es wichtig, Sicherheit zu haben, dass auch in den Randbereichen des verfügbaren Scanfelds die Scankomponenten präzise arbeiten und Soll/Ist-Abweichungen der Fokusposition, wenn überhaupt, nur in tolerablen Maßen auftreten. Zweckmäßigerweise ist daher bei einer bevorzugten Ausführungsform mindestens eine Teilanzahl der Soll-Fokuspositionen am Rand eines gegebenen maximalen Scanfelds angeordnet. Es ist sogar möglich, dass alle Soll-Fokuspositionen am Rand des maximal möglichen Scanfelds angeordnet sind.

Die Soll-Fokuspositionen können mindestens eine Gruppe von Fokuspositionen umfassen, welche in einer zur Strahlungsausbreitungsrichtung orthogonalen Transversalebene verteilt angeordnet sind. Dabei können die Soll-Fokuspositionen mindestens eine Gruppe von Fokuspositionen umfassen, welche in der Transversalebene längs einer Kreislinie verteilt angeordnet sind, insbesondere in gleichmäßigen Winkelabständen. Beispielsweise kann es genügen, nur einige wenige Soll-Fokuspositionen längs der Kreislinie festzulegen, etwa eine Soll-Fokusposition pro Quadrant.

Im Fall longitudinaler Scan-Fähigkeit der Scankomponenten kann zumindest ein Teil der Soll-Fokuspositionen in Strahlungsausbreitungsrichtung verteilt angeordnet sein. Insbesondere ist es vorstellbar, dass die Soll-Fokuspositionen mehrere Gruppen von Fokuspositionen umfassen, welche in verschiedenen zur Strahlungsausbreitungsrichtung orthogonalen Transversalebenen verteilt angeordnet sind. Dabei kann beispielsweise eine erste Gruppe von Soll-Fokuspositionen in einer ersten Transversalebene liegen, welche das verfügbare Scanfeld longitudinal in einer ersten Richtung begrenzt. Eine zweite Gruppe von Soll-Fokuspositionen kann in einer zweiten Transversalebene liegen, welche das verfügbare Scanfeld longitudinal in der anderen Richtung begrenzt. Gewünschtenfalls können weitere Soll-Fokuspositionen in einer oder mehreren transversalen Zwischenebenen liegen, welche zwischen den beiden endseitigen Transversalebenen der ersten und der zweiten Gruppe von Soll-Fokuspositionen liegen. Alternativ oder zusätzlich können die Soll-Fokuspositionen eine Gruppe von Fokuspositionen umfassen, welche längs einer Schraubenlinie verteilt angeordnet sind, deren Schraubenachse parallel zur Strahlungsausbreitungsrichtung verläuft.

Um ein Maß für die Positionierpräzision der Scankomponenten zu gewinnen, kann die Steueranordnung dazu eingerichtet sein, Abweichungen zwischen den Soll-Fokuspositionen und den Ist-Fokuspositionen zu ermitteln und die ermittelten Abweichungen mit mindestens einer vorgegebenen Abweichungsschwelle zu vergleichen. Besonders dann, wenn die Anzahl der durch das Test-Scanmuster vorgegebenen zu testenden Soll-Fokuspositionen vergleichsweise gering ist, beispielsweise im niedrigen zweistelligen Bereich, kann die Forderung bestehen, dass keine Positionierfehler auftreten dürfen, also sämtliche Soll/Ist-Abweichungen innerhalb der durch die mindestens eine Abweichungsschwelle gegebenen Grenzen liegen müssen. In anderen Fällen kann dagegen eine gewisse Anzahl von Positionierfehlern tolerabel sein. Jedenfalls ist in einer bevorzugten Ausgestaltung der Erfindung eine Höchstzahl für diejenigen Fälle vorgegeben, in denen die ermittelten Abweichungen eine zugehörige Abweichungsschwelle übersteigen. Diese Höchstzahl kann entweder Null sein oder ein von Null verschiedener Wert sein. Bevorzugt ist die Steueranordnung dazu eingerichtet, einen Betrieb der Lasereinrichtung mit eingeschalteter Laserquelle nur dann freizugeben, wenn nur höchstens so viele ermittelte Abweichungen eine zugehörige Abweichungsschwelle übersteigen, wie durch die Höchstzahl vorgegeben ist. Andernfalls wird die Steueranordnung die Lasereinrichtung gegen einen Betrieb mit eingeschalteter Laserquelle sperren, so dass keine Laserbearbeitung möglich ist. Bei erfolgloser Durchführung des Testbetriebslaufs wird die Steueranordnung dementsprechend in einen Sperrmodus eintreten, der beispielsweise so ausgestaltet sein kann, dass ihn die Steueranordnung nur nach erfolgreicher Durchführung eines weiteren Testbetriebslaufs der Scankomponenten verlassen kann.

Die Erfindung wird durch die Ansprüche definiert und nachstehend anhand der beigefügten Zeichnungen näher erläutert. Es stellen dar:
- Fig. 1: in schematischer Blockdarstellung Elemente einer Lasereinrichtung nach einem Ausführungsbeispiel, und
- Fig. 2: ein Beispiel für ein aus diskreten Soll-Fokuspositionen bestehendes Test-Scanmuster für die Lasereinrichtung der Fig. 1.

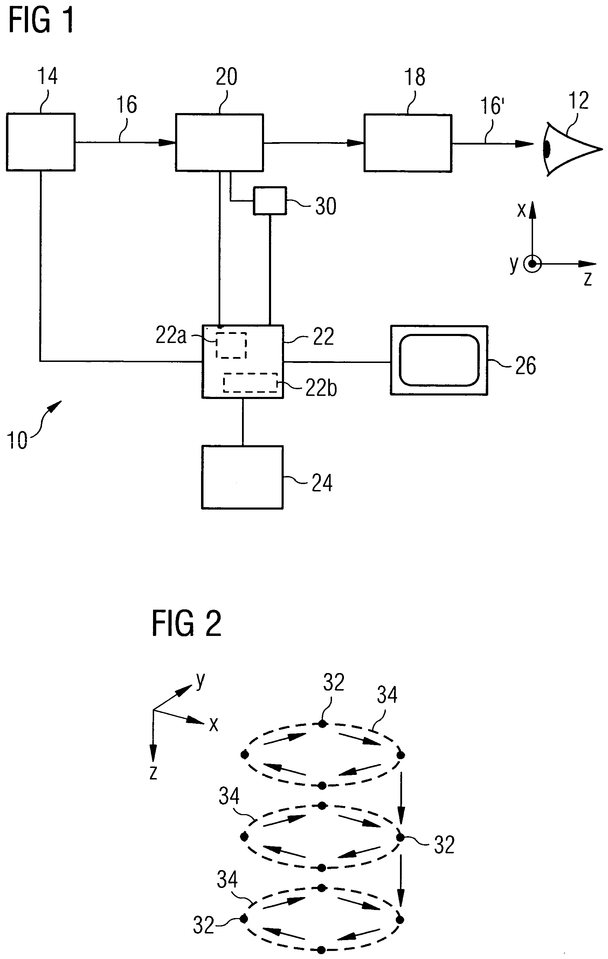

Die Lasereinrichtung der Fig. 1 - dort allgemein mit 10 bezeichnet - dient zur schneidenden Bearbeitung eines im Beispielfall als humanes Auge 12 gezeigten Objekts mittels ultrakurzpulsiger fokussierter Laserstrahlung. Ultrakurzpulsig meint hier Pulsdauern im Bereich von Femtosekunden bis höchstens einstelligen Pikosekunden. Der für die Schneidbearbeitung genutzte Effekt ist der sogenannte laserinduzierte optische Durchbruch, der zu einer Fotodisruption innerhalb des bearbeiteten Materials (hier Augengewebe) führt. Durch Nebeneinandersetzen einer Vielzahl solcher Fotodisruptionen können vielfältige Schnittfiguren in dem Auge 12 und dort vor allem in der Kornea erzeugt werden.

Die Lasereinrichtung 10 umfasst eine Fs-Laserquelle 14, die einen Laserstrahl 16 bereitstellt, welcher nach Durchlauf einer optischen Wegstrecke, längs der verschiedene Elemente zur Strahlführung und -formung angeordnet sind, als fokussierter Laserstrahl 16' auf das Auge 12 fällt. Die erwähnten Elemente zur Strahlführung und -formung umfassen eine beispielsweise von einem F-Theta-Objektiv gebildete Fokussieroptik 18 sowie hier schematisch durch einen einzigen Block angedeutete Scankomponenten 20.

Es ist hervorzuheben, dass die Darstellung der Fokussieroptik 18 und der Scankomponenten 20 in Fig. 1 als getrennte Blöcke allein zum Zweck der besseren Anschauung dient. Es ist ohne weiteres vorstellbar, dass ein Teil der für die Fokussierung des Laserstrahls 16 verantwortlichen optischen Bauteile auch Scanfunktionalität übernehmen kann. So ist es beispielsweise nicht ausgeschlossen, dass eine oder mehrere in der Fokussieroptik 18 enthaltenen Linsen oder sogar die Fokussieroptik 18 als Ganzes zum Zwecke der longitudinalen Positionierung des Strahlfokus in Strahlausbreitungsrichtung verstellbar sind. Gleichwohl sind in einer bevorzugten Ausgestaltung die für das Scannen des Laserstrahls 16 dienenden optischen Bauteile von den zur Fokussierung des Laserstrahls 16 dienenden optischen Bauteilen separiert und infolgedessen außerhalb der Fokussieroptik 18 angeordnet.

Beispielsweise können die Scankomponenten zum transversalen Scannen des Laserstrahls 16 ein Paar drehbar angeordneter Ablenkspiegel, deren Drehachsen senkrecht zueinander liegen, sowie in Zuordnung zu jedem der Ablenkspiegel einen individuell steuerbaren Galvanometerantrieb umfassen. Solche galvanometrisch betätigten Ablenkspiegel sind in der Fachwelt an sich bekannt; es bedarf daher an dieser Stelle keiner näheren Erläuterung hierzu.

Zum longitudinalen Scannen des Strahlfokus können die Scankomponenten 20 beispielsweise eine als Teil einer Strahlaufweitungsoptik (nicht näher dargestellt) vorgesehene Linse nutzen, die zum Zwecke der Divergenzvariation des Laserstrahls 16 in Strahlausbreitungsrichtung verstellbar angeordnet ist oder hinsichtlich ihrer Brechkraft justierbar ist. Ein zugehöriger Steller in Form eines Linearantriebs oder einer steuerbaren Spannungsquelle kann dann ebenfalls Teil der Scankomponenten 20 sein.

In einer Mindestausstattung der Lasereinrichtung 10 sind die Scankomponenten 20 jedenfalls zum transversalen Scannen des Laserstrahls 16 ausgelegt. In einer bevorzugten Ausgestaltung sind die Scankomponenten 20 zusätzlich zum longitudinalen Scannen eingerichtet. Es versteht sich im Übrigen, dass neben den erwähnten beispielhaften Ausgestaltungen der Scankomponenten andere Wirkprinzipien zum Einsatz kommen können, die ein transversales oder/und longitudinales Scannen ermöglichen, z.B. eine gesteuerte Strahlablenkung in einem elektrooptischen Kristall oder eine Divergenzbeeinflussung des Laserstrahls durch Verformung eines im Ausbreitungsweg des Laserstrahls 16 angeordneten optischen Spiegels.

Die Lasereinrichtung 10 enthält zudem eine prozessorbasierte Steueranordnung 22 zur Steuerung des Betriebs der Lasereinrichtung. Die Steueranordnung 22 ist programmgesteuert; in einer Speicheranordnung 24 ist das Steuerprogramm der Steueranordnung 22 abgelegt.

Wenngleich die Steueranordnung 22 in Fig. 1 durch einen einzelnen Block dargestellt ist, versteht es sich, dass ihre Steuerfunktionen auf verschiedene Steuermodule aufgeteilt sein können, die auf gesonderten Steuerplatinen in unterschiedliche Baumodule eingebaut sein können. So kann die Steueranordnung 22 beispielsweise ein gestrichelt bei 22a eingezeichnetes Scan-Steuermodul umfassen, das für die Steuerung der Scankomponenten 20 verantwortlich ist und zusammen mit diesen - oder zusammen mit zumindest einem Teil der Scankomponenten 20 - in einen als gesondertes Bauteil vormontierten Scanner integriert ist. Die verbleibenden Steuerfunktionen der Steueranordnung 22 können beispielsweise in einem baulich außerhalb dieses Scanners gelegenen Zentral-Steuermodul 22b (ebenfalls gestrichelt angedeutet) zusammengefasst sein, das unter anderem für die Synchronisation des Betriebs der Laserquelle 14 und des Betriebs der Scankomponenten 20 verantwortlich ist und dementsprechende Steuerbefehle an das Scan-Steuermodul 22a schicken kann, um einen Scanvorgang zu starten. Die konkreten Stelloperationen zur Einstellung der Scankomponenten 20 werden dann von dem Scan-Steuermodul 22a nach Maßgabe geeigneter Scandaten gesteuert, welche zuvor in das Scan-Steuermodul geladen wurden und ein auszuführendes Scanmuster definieren.

Entsprechend der möglichen Aufteilung der Steueranordnung 22 in gesonderte Steuermodule können auch die Speicheranordnung 24 in gesonderte Speichermodule sowie das erwähnte Steuerprogramm in gesonderte Programmmodule aufgeteilt sein, die ihrerseits in verschiedenen Speichermodulen gespeichert sein können. Beispielsweise kann ein Speichermodul gemeinsam mit dem Scan-Steuermodul 22 in den erwähnten Scanner integriert sein und solche Programmteile speichern, die für die Steuerung der Scankomponenten 20 nötig sind. Ein oder mehrere weitere Speichermodule können dagegen dem Zentral-Steuermodul 22b zugeordnet sein und die übrigen Programmteile des Steuerprogramms speichern.

Mit der Steueranordnung 22 ist ferner eine Ausgabeeinheit 26 in Form eines Monitors verbunden, auf dem später noch zu erläuternde Testergebnisse ausgegeben werden können, die im Rahmen eines Testbetriebslaufs der Lasereinrichtung 10 gewonnen werden. Wenngleich in Fig. 1 nicht näher dargestellt, kann alternativ oder zusätzlich zu dem Monitor 26 ein Drucker an die Steueranordnung 22 angeschlossen sein, um die erwähnten Testergebnisse in gedruckter Form auszugeben.

In Fig. 1 ist zudem ein dreiachsiges Koordinatenkreuz eingezeichnet, das gemäß üblicher Notation eine zur Strahlungsausbreitungsrichtung des Laserstrahls 16 orthogonale x-y-Transversalebene aufspannt, während die z-Achse die Längsrichtung der Strahlausbreitung definiert.

Die Steueranordnung 22 ist dazu eingerichtet, bei abgeschalteter Laserquelle 14 einen Testbetriebslauf durchzuführen, bei dem die Scankomponenten 20 oder zumindest ein Teil derselben nach Maßgabe eines vorgegebenen Test-Scanmusters gesteuert werden. Dieser Trockenlauf, bei dem keine Laserstrahlung von der Laserquelle 14 abgegeben wird, soll eine Positionskontrolle ermöglichen, durch die sichergestellt werden soll, dass der gesamte Bereich, in dem der Strahlfokus in x-, y- und gegebenenfalls z-Richtung nominell eingestellt werden kann, mit bestmöglicher Präzision angefahren werden kann. Insbesondere soll die Positionskontrolle eine Überprüfung etwaiger Soll/Ist-Abweichungen der Fokusposition im gesamten Scanbereich ermöglichen. Der maximale Scanbereich, der im vorliegenden Fall unter der Annahme sowohl transversaler als auch longitudinaler Scan-Fähigkeit ein dreidimensionaler Raum ist, wird hier auch als verfügbares Scanfeld bezeichnet.

Schematisch als einzelner Block angedeutete Messkomponenten 30 sind dazu vorgesehen, den tatsächlichen Einstellzustand wenigstens eines Teils der Scankomponenten 20 messtechnisch zu erfassen und entsprechende Messwerte an die Steueranordnung 22 zu liefern. Diese kann aus den gelieferten Messwerten Werte für die Ist-Position des Strahlfokus berechnen. Beispielsweise können zur Erfassung der Ist-Position eines in den Scankomponenten 20 enthaltenen drehbaren Ablenkspiegels die Messkomponenten 30 einen Positionsdetektor umfassen, wie er in EP 1 295 090 B1 gezeigt und beschrieben ist.

Die berechneten Ist-Fokuspositionen kann die Steueranordnung 22 in der Speicheranordnung 24 in Zuordnung zu zugehörigen Soll-Fokuspositionen abspeichern. Bei einer Schneidbearbeitung des Auges 12 sind die Soll-Fokuspositionen beispielsweise tabellarisch durch Angabe der jeweiligen x-, y- und z-Werte vorgegeben. Nach Abschluss der Schneidbearbeitung des Auges 12 kann die Steueranordnung 22 die zwischenzeitlich ermittelten Ist-Werte der Fokusposition mit den zugehörigen SollWerten vergleichen und entsprechende Informationen über den Monitor 26 oder einen angeschlossenen Drucker ausgeben. Der Operateur oder ein automatisch arbeitendes Auswerteprogramm kann anhand der Ergebnisse der Soll/Ist-Vergleiche feststellen, ob das Scansystem im gesamten verfügbaren Scanraum mit der geforderten hohen Präzision arbeitet.

Der zuvor erwähnte Testbetriebslauf ist vor allem (aber nicht nur) dann zweckmäßig, wenn während des Scanbetriebs der Lasereinrichtung 10 zwar mittels der Messkomponenten 30 Messwerte für die Ist-Fokusposition aufgenommen und abgespeichert werden können, die Lasereinrichtung 10 aber nicht dazu eingerichtet ist, die Ist-Fokuspositionen während des Scanbetriebs im Hinblick auf Abweichungen von den zugehörigen Soll-Fokuspositionen auszuwerten und gegebenenfalls korrigierend einzugreifen, falls unzulässig große Abweichungen auftreten. Solche Gegebenheiten können beispielsweise vorliegen, wenn die Soll- und Ist-Daten der Fokuspositionen während eines Scanvorgangs nicht von dem Scan-Steuermodul 22a zum Zentral-Steuermodul 22b übertragen werden können, sondern das Zentral-Steuermodul 22b erst nach Abschluss des Scanvorgangs Zugriff auf diese Daten bekommt. Der Testbetriebslauf ermöglicht so eine Vorabprüfung der Positionierqualität der Scankomponenten 20. Je nach Ergebnis des Testbetriebslaufs kann sich herausstellen, dass die Scanpräzision im verfügbaren Scanfeld ausreichend hoch ist, um eine geplante Augenbearbeitung durchzuführen, oder es können Maßnahmen wie ein Austausch zumindest eines Teils der Scankomponenten nötig sein, sollte sich die Scangenauigkeit als unzureichend erweisen.

Der Testbetriebslauf kann durch die Steueranordnung 22 beispielsweise in Reaktion auf einen benutzerseitig eingegebenen Startbefehl zur Durchführung einer Laserbearbeitung (etwa zur Erzeugung eines Flap-Schnitts im Rahmen einer LASIK-Operation des Auges 12) durchgeführt werden, wobei die Steueranordnung die Laserbearbeitung nur dann beginnt, wenn zuvor der Testbetriebslauf erfolgreich durchgeführt wurde. Ansonsten kann sie beispielsweise eine geeignete Warnmeldung auf dem Monitor 26 ausgeben, die den Operateur darauf hinweist, dass der Testbetriebslauf erfolglos war und deswegen die geplante Laserbearbeitung nicht durchgeführt werden kann.

Zur Erläuterung des im Rahmen des Testbetriebslaufs abgearbeiteten Test-Scanmusters wird nun zusätzlich auf Fig. 2 verwiesen. Das Test-Scanmuster setzt sich aus einer Mehrzahl diskreter Soll-Fokuspositionen 32 zusammen, deren x-, y- und z-Koordinatenwerte zuvor in die Steueranordnung 22 und dort insbesondere in das Scan-Steuermodul 22a geladen wurden. Im Rahmen des Testbetriebslaufs werden die Scankomponenten 20 so eingestellt, dass in rascher Aufeinanderfolge die vorgegebenen Soll-Fokuspositionen angefahren werden. Die Scankomponenten 20 werden dabei solange kontinuierlich gesteuert, bis eine Soll-Fokusposition erreicht ist. Die Steuerung der Scankomponenten 20 stoppt dann für eine kurze Zeit, bevor die Scankomponenten 20 erneut justiert werden, um die nächste Soll-Fokusposition anzufahren. Insoweit ist der Justiervorgang der Scankomponenten 20 ein schrittweiser Prozess, im Unterschied zu einem kontinuierlichen Abfahren einer vorgegebenen Bahn.

Im gezeigten Beispielfall der Fig. 2 sind die Soll-Fokuspositionen 32 auf mehrere (hier drei) Gruppen aufgeteilt, die jeweils einer x, y-Transversalebene zugeordnet sind. In jeder Transversalebene sind die Soll-Fokuspositionen 32 der betreffenden Gruppe längs einer gedachten Kreislinie 34 in regelmäßigen Winkelabständen verteilt angeordnet. Die gedachten Kreislinien 34 sind in Fig. 2 gestrichelt angedeutet. Insgesamt sind im Beispielfall der Fig. 2 pro Kreislinie 34 vier Soll-Fokuspositionen 32 vorgegeben, d.h. eine Soll-Fokusposition pro Quadrant. Es versteht sich, dass die Anzahl der Soll-Fokuspositionen 32 pro Transversalebene hiervon abweichen kann, insbesondere auch größer sein kann. Ebenso versteht es sich, dass in Abweichung vom gezeigten Beispielfall, wo die Anzahl der Soll-Fokuspositionen 32 in jeder Transversalebene gleich ist, diese Anzahl für verschiedene Transversalebenen unterschiedlich sein kann.

Die Soll-Fokuspositionen 32 liegen im Beispielfall der Fig. 2 in den verschiedenen Transversalebenen longitudinal übereinander, d.h. sie sind in z-Richtung miteinander in Überdeckung. Auch dies ist keine Notwendigkeit; es ist ohne weiteres denkbar, von Transversalebene zu Transversalebene ein zueinander winkelversetztes Anordnungsbild der Soll-Fokuspositionen 32 zu wählen.

Die zeitliche Reihenfolge, in welcher die Soll-Fokuspositionen 32 angefahren werden, ist an sich beliebig wählbar. Zweckmäßig ist es, wenn zunächst alle Soll-Fokuspositionen in einer Transversalebene angefahren werden, bevor zur nächsten Transversalebene gewechselt wird. Innerhalb einer Transversalebene können die Soll-Fokuspositionen beispielsweise einzeln nacheinander längs der betreffenden Kreislinie 34 angefahren werden.

Unter der Annahme eines kreiszylindrischen verfügbaren Scanfelds (d.h. eines Scanfelds, dessen transversale Außengrenze kreisförmig ist) können die Kreislinien 34 beispielsweise an der Außengrenze dieses verfügbaren Scanfelds verlaufen, so dass die Soll-Fokuspositionen 32 vor allem dazu dienen, die Scanpräzision an den Außengrenzen des verfügbaren Scanfelds zu testen. Es ist gleichwohl vorstellbar, die Soll-Fokuspositionen 32 innerhalb des verfügbaren Scanfelds im Abstand von dessen Außengrenzen anzuordnen, wenn eine Positionskontrolle der Innenbereiche des verfügbaren Scanfelds erwünscht ist.

Sobald eine der Soll-Fokuspositionen 32 angefahren ist und ein kurzzeitiger Steuerstopp der Scankomponenten 20 stattfindet, wird mittels der Messkomponenten 30 die Ist-Fokusposition erfasst und in Zuordnung zu der betreffenden Soll-Fokusposition 32 abgespeichert. Die Messung findet also im Stillstand des Scansystems statt, d.h. während eines Stopps der Scanbewegung. Um Einschwingvorgänge im Messergebnis zu eliminieren, wird nach Erreichen einer Soll-Fokusposition vorzugsweise noch eine gewisse Zeit abgewartet, bevor die Messung durchgeführt wird, beispielweise einige Millisekunden.

Sodann wird die Scanbewegung fortgesetzt und die nächste Soll-Fokusposition angefahren. Dies wird nacheinander für alle Soll-Fokuspositionen 32 wiederholt. Es kann genügen, für die Soll-Fokuspositionen 32 oder/und die Ist-Fokuspositionen nur eine oder zwei Koordinaten statt alle drei Koordinaten des xyz-Koordinatensystems zu berücksichtigen und zu speichern. Für die Soll-Fokuspositionen 32 des Test-Scanmusters gemäß Fig. 2 kann es beispielsweise genügen, die Soll-Fokuspositionen oder/und die Ist-Fokuspositionen nur durch den z-Koordinatenwert und einen transversalen Koordinatenwert darzustellen, etwa den x-Koordinatenwert. Soweit die Lasereinrichtung 10 dies ermöglicht, kann es jedoch wünschenswert sein, für sämtliche Soll-Fokuspositionen 32 und zugeordnete Ist-Fokuspositionen alle drei Koordinatenwerte zu registrieren.

Nach Ausführung des Test-Scanmusters nimmt die Steueranordnung 22 eine Analyse der erfassten Ist-Fokuspositionen im Hinblick auf Abweichungen von den Soll-Fokuspositionen vor. Hierbei vergleicht sie die gefundenen Abweichungen mit mindestens einer vorgegebenen Schwelle. Beispielsweise kann die Steueranordnung 22 die Abweichungen zwischen den Soll-Fokuspositionen und den Ist-Fokuspositionen getrennt für jede Koordinatenachse einzeln ermitteln und mit einer zugeordneten Abweichungsschwelle vergleichen. Die anzuwendende Abweichungsschwelle kann für die Koordinatenachsen gleich sein oder für unterschiedliche Koordinatenachsen unterschiedlich sein. Gewünschtenfalls kann die Steueranordnung 22 zusätzlich eine Gesamtabweichung aus den längs der einzelnen Koordinatenachsen gefundenen Abweichungen ermitteln, etwa nach Art des euklidischen Abstands. Auch die so gefundene Gesamtabweichung kann sie mit einer zugeordneten Abweichungsschwelle vergleichen. Es versteht sich, dass jede Abweichungsschwelle entweder fest vorgegeben sein kann oder vom Anwender über eine nicht näher dargestellte, mit der Steueranordnung 22 verbundene Eingabeeinheit wählbar sein kann.

Im besten Fall liegen alle Ist-Fokuspositionen innerhalb der gegebenen Abweichungsgrenzen. Sobald mindestens eine Ist-Fokusposition um ein unzulässig großes Maß von der zugeordneten Soll-Fokusposition 32 abweicht, veranlasst die Steueranordnung 22 die Ausgabe eines Warnhinweises auf dem Monitor 26. Der Warnhinweis teilt dem Anwender mit, dass die Scanpräzision nicht ausreichend ist. Als weitere Reaktion kann die Steueranordnung 22 in einen Sperrmodus eintreten, der einen Betrieb der Lasereinrichtung 10 mit eingeschalteter Laserquelle 14 unterbindet. Erst nach erfolgreicher Durchführung eines weiteren Testbetriebslaufs, bei dem alle erfassten Ist-Fokuspositionen innerhalb der gegebenen Abweichungsgrenzen geblieben sind, gelangt die Steueranordnung 22 wieder aus dem Sperrmodus heraus und ermöglicht den Betrieb der Lasereinrichtung 10 mit eingeschalteter Laserquelle 14.

Alternativ oder zusätzlich kann die Steueranordnung 22 dazu eingerichtet sein, die erfassten Ist-Fokuspositionen zusammen mit den Soll-Fokuspositionen oder/und die ermittelten Soll/Ist-Abweichungen ohne weitere Analyse auf dem Monitor 26 oder in anderer Form auszugeben. In diesem Fall kann die Bewertung des Testergebnisses dem Anwender überlassen werden.

Der soweit erläuterte Testbetriebslauf dient der Prüfung des statischen Verhaltens des Scansystems, insbesondere der Präzision der Positionsfindung, und zusätzlich der Prüfung, ob alle Signalwege noch korrekt arbeiten, etwa eine analoge Signalleitung von einer Treiber-Leiterplatte zu einem Scannerservo sowie ein Rückkanal von einem Encoder (Teil der Messkomponenten 30) zur Steueranordnung 22.

## Patentansprüche

1. Lasereinrichtung, insbesondere für die ophthalmologische Laserchirurgie, umfassend
- eine Laserquelle (14) zur Bereitstellung von Laserstrahlung,
- steuerbare Scankomponenten (20) zur Einstellung einer Fokusposition der Laserstrahlung,
- Messkomponenten (30) zur Erfassung von Informationen, welche für eine Ist-Position des Strahlungsfokus repräsentativ sind,
- eine die Laserquelle (14) und die Scankomponenten (20) steuernde Steueranordnung (22), welche dazu eingerichtet ist, die Durchführung eines Testbetriebslaufs mindestens eines Teils der Scankomponenten bei abgeschalteter Laserquelle nach Maßgabe eines vorgegebenen Test-Scanmusters zu bewirken, wobei das Test-Scanmuster eine Mehrzahl nacheinander anzufahrender diskreter Soll-Fokuspositionen (32) repräsentiert und die Steueranordnung (22) dazu eingerichtet ist, bei jeder der Soll-Fokuspositionen die Scanbewegung der Scankomponenten (20) anzuhalten und in Zuordnung zu jeder der Soll-Fokuspositionen jeweils eine Ist-Fokusposition auf Grundlage der erfassten Informationen der Messkomponenten (30) zu ermitteln, wobei die Steueranordnung (22) dazu eingerichtet ist, Abweichungen zwischen den Soll-Fokuspositionen (32) und den Ist-Fokuspositionen zu ermitteln, **dadurch gekennzeichnet,**
**dass** die Steuereinrichtung weiterhin eingerichtet ist,
die ermittelten Abweichungen mit mindestens einer vorgegebenen Abweichungsschwelle zu vergleichen und einen Betrieb der Lasereinrichtung mit eingeschalteter Laserquelle (14) nur dann freizugeben, wenn nicht mehr als eine vorgegebene Höchstzahl der ermittelten Abweichungen eine zugehörige Abweichungsschwelle übersteigt.

2. Lasereinrichtung nach Anspruch 1, wobei die Soll-Fokuspositionen (32) mindestens eine Gruppe von Fokuspositionen umfassen, welche in einer zur Strahlungsausbreitungsrichtung orthogonalen Transversalebene verteilt angeordnet sind.

3. Lasereinrichtung nach Anspruch 2, wobei die Soll-Fokuspositionen (32) mindestens eine Gruppe von Fokuspositionen umfassen, welche in der Transversalebene längs einer Kreislinie (34) vorzugsweise in gleichmäßigen Winkelabständen verteilt angeordnet sind.

4. Lasereinrichtung nach einem der Ansprüche 1 bis 3, wobei zumindest ein Teil der Soll-Fokuspositionen (32) in Strahlungsausbreitungsrichtung verteilt angeordnet ist.

5. Lasereinrichtung nach Anspruch 4, wobei die Soll-Fokuspositionen (32) mehrere Gruppen von Fokuspositionen umfassen, welche in verschiedenen zur Strahlungsausbreitungsrichtung orthogonalen Transversalebenen verteilt angeordnet sind.

6. Lasereinrichtung nach einem der Ansprüche 1 bis 5, wobei mindestens eine Teilanzahl der Soll-Fokuspositionen (32), gewünschtenfalls alle Soll-Fokuspositionen, am Rand eines gegebenen maximalen Scanfelds angeordnet sind.

7. Lasereinrichtung nach einem der vorhergehenden Ansprüche, wobei die Steueranordnung (22) dazu eingerichtet ist, einen Betrieb der Lasereinrichtung mit eingeschalteter Laserquelle (14) nur dann freizugeben, wenn keine der ermittelten Abweichungen die zugehörige Abweichungsschwelle übersteigt.

## Claims

1. Laser apparatus, in particular for ophthalmological laser surgery, comprising
- a laser source (14) for providing laser radiation,
- controllable scan components (20) for setting a focal position of the laser radiation,
- measuring components (30) for acquiring information representative of an actual position of the radiation focus,
- a control arrangement (22) controlling the laser source (14) and the scan components (20), said control arrangement being configured to effect a test operation of at least some of the scan components being carried out when the laser source is switched off, according to the stipulation of a predetermined test scan pattern,
wherein the test scan pattern represents a plurality of discrete intended focal positions (32) to be approached in sequence and the control arrangement (22) is configured to stop the scan movement of the scan components (20) at each intended focal position and establish, assigned to each one of the intended focal positions, an actual focal position in each case on the basis of the acquired information from the measuring components (30), wherein the control arrangement (22) is configured to establish deviations between the intended focal positions (32) and the actual focal positions,
**characterized in that** the control apparatus is furthermore configured
to compare the established deviations with at least one predetermined deviation threshold and only to release operation of the laser apparatus with a switched-on laser source (14) if not more than a predetermined maximum number of the established deviations exceeds an associated deviation threshold.

2. Laser apparatus according to Claim 1, wherein the intended focal positions (32) comprise at least one group of focal positions which are arranged distributed in a transversal plane orthogonal to the radiation propagation direction.

3. Laser apparatus according to Claim 2, wherein the intended focal positions (32) comprise at least one group of focal positions which are arranged along a circular line (34), preferably distributed at regular angular intervals, in the transversal plane.

4. Laser apparatus according to one of Claims 1 to 3, wherein at least some of the intended focal positions (32) are arranged distributed in the radiation propagation direction.

5. Laser apparatus according to Claim 4, wherein the intended focal positions (32) comprise a plurality of groups of focal positions which are arranged distributed in various transversal planes orthogonal to the radiation propagation direction.

6. Laser apparatus according to one of Claims 1 to 5, wherein at least a subset of the intended focal positions (32), if desired all intended focal positions, is/are arranged on the edge of a given maximum scan field.

7. Laser apparatus according to one of the preceding claims, wherein the control arrangement (22) is configured only to release operation of the laser apparatus with a switched-on laser source (14) if none of the established deviations exceed the associated deviation threshold.

## Revendications

1. Dispositif au laser, notamment destiné à la chirurgie ophtalmologique au laser, comprenant
- une source laser (14) pour la mise à disposition d'un rayonnement laser,
- des composants de balayage (20) susceptibles d'être commandés pour régler une position de focalisation du rayonnement laser,
- des composants de mesure (30) destinés à détecter des informations, lesquelles sont représentatives pour une position réelle de focalisation du rayonnement,
- un agencement de commande (22) commandant la source laser (14) et les composants de balayage (20), lequel est agencé pour provoquer la réalisation selon un modèle de balayage de test prédéfini d'un mode de test pour au moins une partie des composants de balayage, lorsque la source laser est désactivée, le modèle de balayage de test représentant une pluralité de positions discrètes de focalisation (32) de consigne qui doivent être successivement abordées et l'agencement de commande (22) étant agencé pour arrêter, à chacune des positions de focalisation de consigne le déplacement en balayage des composants de balayage (20) et en association avec chacune des positions de focalisation de consigne, pour déterminer chaque fois une position de focalisation réelle sur la base des informations détectées par les composants de mesure (30), l'agencement de commande (22) étant agencé pour déterminer des divergences entre les positions de focalisation (32) de consigne et les positions de focalisation réelles, **caractérisé en ce que** le dispositif de commande est agencé en outre pour comparer les divergences déterminées avec au moins un seuil de divergences prédéfini et pour ne valider un fonctionnement du dispositif au laser avec la source laser (14) activée que si pas plus qu'un nombre maximum prédéfini des divergences déterminées ne dépasse un seuil de divergences correspondant.

2. Dispositif au laser selon la revendication 1, les positions de focalisation (32) de consigne comprenant au moins un groupe de positions de focalisation qui sont placées en étant distribuées dans un plan transversal orthogonal par rapport à la direction de propagation du rayonnement.

3. Dispositif au laser selon la revendication 2, les positions de focalisation (32) de consigne comprenant au moins un groupe de positions de focalisation qui sont placées en étant distribuées dans le plan transversal le long d'une ligne circulaire (34), de préférence avec des écarts angulaires réguliers.

4. Dispositif au laser selon l'une quelconque des revendications 1 à 3, au moins une partie des positions de focalisation (32) de consigne étant placée en étant distribuée dans la direction de propagation du rayonnement.

5. Dispositif au laser selon la revendication 4, les positions de focalisation (32) de consigne comprenant plusieurs groupes de positions de focalisation, lesquels sont placés en étant distribués dans différents plans transversaux orthogonaux par rapport à la direction de propagation du rayonnement.

6. Dispositif au laser selon l'une quelconque des revendications 1 à 5, au moins un nombre partiel des positions de focalisation (32) de consigne, si on le souhaite, toutes les positions de focalisation de consigne, étant placées sur le bord d'un champ de balayage maximal donné.

7. Dispositif au laser selon l'une quelconque des revendications précédentes, l'agencement de commande (22) étant agencé pour ne valider un fonctionnement du dispositif au laser avec la source laser (14) activée que si aucune des divergences détectées ne dépasse le seuil de divergences correspondant.
